# EUROPÄISCHE PATENTANMELDUNG

(11) **EP 2 275 818 A2**
(43) Veröffentlichungstag der Anmeldung: **19.01.2011**
(21) Anmeldenummer: 10183478.6
(22) Anmeldetag: 11.08.1995
(51) Int. Cl.: G01N 33/576, C12N 15/51, C07K 14/18

(54) **Verfahren zur Früherkennung der Serokonversion eines gegen Hepatitis C-Virus gerichteten Antikörpers**

(30) Priorität: 12.08.1994 DE 4428705
(62) Teilanmeldung aus: 08016723.2
(71) Anmelder: Roche Diagnostics GmbH, 68305 Mannheim (DE)
(72) Erfinder: Seidel, Christoph, 82362 Weilheim (DE); Wienhues, Ursula-Henrike, 82152 Krailling (DE); Schmitt, Urban, 82386 Oberhausen (DE); Motz, Manfred, 80689 München (DE); Wiedmann, Michael, 82377 Penzberg (DE); Upmeier, Barbara, 82393 Iffeldorf (DE); Soutchek, Erwin, 82335 Berg (DE)
(74) Vertreter: Koch, Andreas

(57) **Zusammenfassung**

Die vorliegende Erfindung betrifft ein Verfahren zur verbesserten Früherkennung der Serokonversion eines gegen Hepatitis C-Virus (HCV) gerichteten Antikörpers in einer Probenflüssigkeit.

## Beschreibung

Die Erfindung betrifft ein Verfahren zur verbesserten Früherkennung der Serokonversion eines gegen Hepatitis C-Virus (HCV) gerichteten Antikörpers in einer Probenflüssigkeit.

Die als Non-A-non-B-Hepatitis bezeichnete Krankheit wird in vielen Fällen durch das Hepatitis C-Virus (HCV) hervorgerufen. Bei HCV handelt es sich um ein einzelsträngiges verkapseltes RNA-Virus, dessen Genom aus etwa 9 bis 10.000 Basen besteht. Von diesem Genom werden Strukturproteine (Kern- und Hüllproteine) sowie Nicht-Strukturproteine kodiert. Die Nicht-Strukturprotein 3 (NS3)-Region von HCV enthält eine Protease und eine Helicase. Die Protease-Aktivität ist im aminoterminalen Drittel der NS3-Region lokalisiert.

In der europäischen Patentanmeldung EP-A-0 318 216 ist eine partielle Nukleotidsequenz von HCV offenbart. Es wird die Verwendung von Nukleinsäurefragmenten oder Polypeptidabschnitten aus HCV zum diagnostischen Nachweis sowie zur therapeutischen Behandlung beansprucht.

EP-A-0 450 931 offenbart die vollständige Nukleotid- und Aminosäuresequenz von HCV. Weiterhin wird eine Kombination von synthetischen HCV-Antigenen beschrieben, umfassend ein erstes HCV-Antigen aus der C-Domäne und mindestens ein weiteres HCV-Antigen aus einer der Nicht-Strukturdomänen NS3, NS4 oder NS5 und der Hülldomäne S. Ein bevorzugtes Antigen aus der Domäne NS3 ist ein mit C33c bezeichnetes Antigen, welches die Aminosäuren 1192 bis 1457 des in Figur 1 von EP-A-0 450 931 dargestellten HCV-Genoms umfasst.

Die internationale Patentanmeldung WO 92/11370 betrifft die Klonierung und Sequenzierung von verschiedenen Polypeptiden aus dem Genom von HCV und die Verwendung dieser Polypeptide ohne Fremdproteinanteile bei Testkits und als Impfstoff. Ein in Zusammenhang mit dieser Anmeldung bei der Deutschen Sammlung für Mikroorganismen und Zellkulturen GmbH, Mascheroder Weg 1 b, 38124 Braunschweig, BRD (DSM) unter der Nummer 6847 hinterlegter Klon NS-3 enthält die genetische Information für ein Polypeptid von 527 Aminosäuren aus der NS3-Region von HCV.

Mori et al. (Jpn. J. Cancer Res. 83 (1992), 264-268) beschreiben den diagnostischen Nachweis einer HCV-Infektion durch Bestimmung viraler Antikörper in Blut unter Verwendung von viralen Proteinen als Anitgene. Diese HCV-Proteine werden als Fusionsproteine mit β-Galactosidase in E.coli exprimiert. Aus der NS3-Region zeigte ein Protein, welches die Aminosäuren 1295 bis 1541 des HCV-Genoms enthielt, die höchste Sensitivität. Nachteilig bei derartigen Fusionsproteinen ist jedoch, dass Kreuzreaktionen mit dem anfusionierten Proteinanteil auftreten können, welche die Spezifität der Nachweisreaktion verringern.

Beim Nachweis von HCV in Blut ist eine hohe Spezifität und Sensitivität des Tests erforderlich. Weiterhin sollte das für den Test verwendete Antigen in hoher Ausbeute exprimierbar und stabil sein. Bisher bekannte Antigene aus dem HCV-Genom weisen Nachteile auf, da sie eine oder mehrere der obigen Anforderungen nicht erfüllen.

Eine der vorliegeden Erfindung zugrunde liegende Aufgabe bestand somit in der Bereitstellung eines Polypeptids aus dem HCV-Genom für ein Verfahren zur verbesserten Früherkennung der Serokonversion eines gegen Hepatitis C-Virus gerichteten Antikörpers, bei dem diese Nachteile des Standes der Technik mindestens teilweise beseitigt sind und das insbesondere verglichen mit bekannten Antigenen eine höhere Spezifität und Sensitivität besitzt sowie in hoher Ausbeute exprimierbar und stabil ist.

Diese Aufgabe wird gelöst durch ein Polypeptid, das Sequenzbereiche aus Hepatitis C-Virus, insbesondere aus der NS3-Region enthält.

Gegenstand der vorliegenden Anmeldung ist somit ein Verfahren zur verbesserten Früherkennung der Serokonversion eines gegen Hepatitis C-Virus gerichteten Antikörpers in einer Probenflüssigkeit , wobei man die Probenflüssigkeit mit mindestens einem Polypeptid, das Sequenzbereiche aus Hepatitis C-Virus, insbesondere aus der NS3-Region von Hepatitis C-Virus, enthält, inkubiert und den Antikörper über eine Bindung mit dem Polypeptid nachweist, wobei man ein Polypeptid aus einem Bereich, der mindestens einen Cysteinrest enthält, verwendet und (a) die Bestimmung des Antikörpers unter reduzierenden Bedingungen durchführt, (b) einen oder mehrere Cysteinreste kovalent modifiziert oder/und (c) einen oder mehrere Cysteinreste durch andere Aminosäuren ersetzt.

Das erfindungsgemäße Verfahren basiert auf der immunologischen Bestimmung eines gegen Hepatitis C-Virus gerichteten Antikörpers in einer Probenflüssigkeit,wobei man die Probenflüssigkeit mit mindestens einem Polypeptid, das Sequenzbereiche aus dem Hepatitis C-Virus, insbesondere aus der NS3-Region von Hepatitis C-Virus enthält, inkubiert und den Antikörper über eine Bindung mit dem Polypeptid nachweist, dadurch gekennzeichnet, dass man ein Polypeptid aus einem Bereich, der mindestens einen Cysteinrest enthält, verwendet und (a) die Bestimmung des Antikörpers unter reduzierenden Bedingungen durchführt, (b) einen oder mehrere Cysteinreste kovalent modifiziert oder/und (c) einen oder mehrere Cysteinreste durch andere Aminosäuren ersetzt.

Im Gegensatz zu anderen antigenen Bereichen aus HCV enthält die NS3-Region eine besonders große Häufung von Cysteinresten. Obwohl das im Verlauf der Virusvermehrung intrazellulär synthetisierte NS3-Protein stabil ist, erwies sich die Verwendung von NS3-Antigenen unter physiologischen Pufferbedingungen, z.B. in immunologischen Testverfahren, als äußerst problematisch, da unter diesen Bedingungen die freien Sulfhydrylgruppen der Cysteinreste leicht oxidieren. Dies führt sowohl zu intra- als auch zu intermolekularen Verletzungen des Antigens, wodurch dessen immunologische Reaktivität deutlich verringert wird.

Durch ein Testformat, bei dem ein oder mehrere NS3-Antigene mit modifizierten Cysteinresten oder/und mit substituierten Cysteinresten eingesetzt werden oder bei dem mild reduzierende Bedingungen, z.B. durch Zusatz eines Sulfhydrylreagenz, vorliegen, konnte nun überraschenderweise die immunologische Reaktivität dieser NS3-Antigene deutlich verbessert werden. Auf diese Weise wird sowohl eine frühzeitige Erkennung der Serokonversion als auch eine deutliche Verstärkung des Messsignals erreicht.

Ein in dem erfindungsgemäßen Verfahren bevorzugt verwendetes Polypeptid ist beispielsweise ein Polypeptid, das aus den Aminosäuren 1207 ± 10 bis 1488 ± 10 eines Hepatitis C-Virus und weniger als 20, vorzugsweise weniger als 15 fremden Aminosäuren besteht. Vorzugsweise enthält das Polypeptid die Aminosäuren 1207 ± 5 bis 1488 ± 5, besonders bevorzugt 1207 ± 2 bis 1488 ± 2 und am meisten bevorzugt 1207 bis 1488 eines Hepatitis C-Virus, wobei sich die Nummerierung der Aminosäurereste auf Fig. 1 von EP-A-0 450 931 bezieht.

Das in dem erfindungsgemäßen Verfahren verwendete Polypeptid kann aus einem beliebigen HCV-Isolat stammen, beispielsweise aus einem HCV-Isolat mit einer Nukleotidsequenz, wie sie in EP-A-0 450 931 beschrieben ist. Vorzugsweise stammt jedoch das Polypeptid aus dem HCV-Isolat, aus dem der in WO 92/11370 beschriebene Klon NS3 stammt, der bei der Deutschen Sammlung von Mikroorganismen und Zellkulturen GmbH(DSM), Mascheroder Weg 1 b, 38124 Braunschweig, unter der Nummer DSM 6847 hinterlegt wurde. Besonders bevorzugt ist das in dem erfindungsgemäßen Verfahren verwendete Polypeptid durch rekombinante Expression des Vektors pUC-D26 erhältlich.

In SEQ ID NO. 1 ist die Aminosäuresequenz eines in dem erfindungsgemäßen Verfahren bevorzugt verwendeten Polypeptids gezeigt. Die Aminosäuren 1-13 sind fremde Aminosäuren. Die Aminosäuren 14-295 stammen aus HCV. Besonders bevorzugt enthält das erfindungsgemäße Polypeptid die Aminosäuren14-295 der in SEQ ID NO. 1 und 2 dargestellten Aminosäuresequenz oder eine dazu mindestens 90 % homologe Aminosäuresequenz.

Des Weiteren kann das in dem erfindungsgemäßen Verfahren verwendete Polypeptid ein wie oben definiertes Polypeptid sein, das mindestens eine Markierungsgruppe trägt. Als Markierungsgruppe kommen alle bekannten Markierungsgruppen in Frage, die in einem Testsystem nachgewiesen werden können, d.h. direkt oder indirekt nachweisbare Markierungsgruppen. Dabei ist unter einer direkt nachweisbaren Markierungsgruppe eine solche Gruppe zu verstehen, die ein direkt nachweisbares Signal erzeugt, z.B. eine radioaktive Gruppe, eine Enzymgruppe, eine Lumineszenzgruppe, ein Metallkomplex etc. Andererseits kann die Markierungsgruppe auch eine indirekt nachweisbare Gruppe sein, z.B. eine Biotin- oder Hapten-Gruppe, die durch Reaktion mit einem geeigneten Bindepartner (Streptavidin, Avidin bzw. Anti-Hapten-Antikörper), der wiederum eine Signal-erzeugende Gruppe trägt, nachweisbar ist. Die Markierungsgruppe kann mit dem Antigen auf bekannte Weise gekoppelt werden, beispielsweise über einen bifunktionellen Spacer. Derartige Verfahren zur Kopplung von Markierungsgruppen an Peptidantigene sind einem Fachmann auf dem Gebiet der Immunologie bekannt und brauchen hier nicht näher beschrieben werden.

Das in dem erfindungsgemäßen Verfahren verwendete Polypeptid enthält vorzugsweise 1 bis 12 und besonders bevorzugt 3 bis 7 Markierungsgruppen.

Des Weiteren kann das in dem erfindungsgemäßen Verfahren verwendete Polypeptid ein wie oben definiertes Polypeptid sein, bei dem eine oder mehrere der aus Cysteinresten stammenden Sulfhydrylgruppen in kovalent modifizierter Form vorliegen. Beispiele für geeignete kovalente Modifizierungsgruppen sind Maleimidodioxaoctylamin (MADOO), N-Methyl-Maleinimid (NMM), Jodessigsäure und Jodacetamid. Durch die kovalente Cysteinmodifikation wird eine besonders hohe spezifische immunologische Reaktivität erzielt, was beispielsweise für die Modifikation NS3 und DTT gezeigt werden konnte.

Besonders bevorzugt erfolgt die kovalente Kopplung von direkt oder indirekt nachweisbaren Markierungsgruppen an die Sulfhydrylgruppen des Polypeptids. Beispiele für SH-reaktive bifunktionelle Linker zur Kopplung an Sulfhydrylgruppen sind Maleimidopropylamin (MP), Maleimidoethylamin (MEA) und Maleimidodioxaoctylamin (MADOO).

Weiterhin kann es in dem erfindungsgemäßen Verfahren bevorzugt sein, ein Polypeptid zu verwenden, bei dem ein oder mehrere Cysteinreste durch andere natürliche oder artifizielle Aminosäuren ersetzt sind. Vorzugsweise werden Cysteinreste durch strukturell analoge α-Aminosäuren, z.B. Serin, oder α-Aminobuttersäure ersetzt. Durch Cysteinsubstitutionen wird eine besonders hohe Stabilität erhalten. Dies konnte insbesondere für NS3 + DTT gezeigt werden.

Das in dem erfindungsgemäßen Verfahren bevorzugt verwendete Polypeptid weist gegenüber bereits bekannten Polypeptiden überraschende Vorteile auf. Gegenüber dem in EP-A-0 450 931 beschriebenen Antigen C33, welches die Aminosäurereste 1192 bis 1457 der HCV-Sequenz enthält, zeigt dieses Polypeptid eine deutlich höhere Spezifität, die sich in einer statistisch signifikant geringeren Anzahl an falsch positiven Resultaten bei negativen Seren zeigt. Gegenüber dem in WO 92/11370 beschriebenen Antigen NS3, welches den Bereich der Aminosäuren 1007 bis 1534 der HCV-Sequenz enthält, zeigt dieses Polypeptid eine deutlich höhere Stabilität unter Testbedingungen. Gegenüber einem Polypeptid, das die Aminosäuren 1227 bis 1528 aus der NS3-Region von HCV enthält, hat dieses Polypeptid den Vorteil einer besseren Expressionseffizienz und einer höheren Sensitivität. Aufgrund dieser Vorteile ist das in dem erfindungsgemäßen Verfahren bevorzugt verwendete Polypeptid allen bisher bekannten HCV-Antigenen aus der NS3-Region deutlich überlegen.

Das in dem erfindungsgemäßen Verfahren verwendete Polypeptid ist durch eine Nukleinsäure kodiert. Ein bevorzugtes Beispiel für eine derartige Nukleinsäure ist die Fremd-DNA-Insertion im Vektor pUC-D26.

In SEQ ID NO. 1 ist auch die Nukleotidsequenz einer für ein im erfindungsgemäßen Verfahren bevorzugt verwendetes Polypeptid kodierenden Nukleinsäure gezeigt, die für das Polypeptid von SEQ ID NO. 1 und 2 kodiert. Die Nukleotide 40-885 kodieren für den aus HCV stammenden Bereich des Polypeptids. Die für ein im erfindungsgemäßen Verfahren bevorzugt verwendetes Polypeptid kodierende Nukleinsäure enthält vorzugsweise (a) die Nukleotide 40-885 der in SEQ ID NO. 1 dargestellte Nukleotidsequenz oder (b) eine der Sequenz aus (a) im Rahmen der Degeneration des genetischen Codes entsprechende Nukleotidsequenz.

Des Weiteren kann die für das im erfindungsgemäßen Verfahren bevorzugt verwendete Polypeptid kodierende Nukleinsäure in einem Vektor vorliegen, wobei der Vektor mindestens eine Kopie einer solchen Nukleinsäure enthält. Der Vektor ist vorzugweise ein prokaryontischer Vektor, d.h. ein zur Propagierung in einer prokaryontischen Wirtszelle geeigneter Vektor. Beispiele für solche Vektoren sind bei Sambrook et al. (Molecular Cloning. A Laboratory Manual, 2nd Ed., Cold Spring Harbor Laboratory Press (1989)), insbesondere in den Kapiteln 1 bis 4 und 17 dargestellt. Besonders bevorzugt ist der Vektor ein zirkuläres Plasmid. Die für das im erfindungsgemäßen Verfahren bevorzugtverwendetePolypeptid kodierende Nukleinsäure befindet sich auf dem Vektor vorzugsweise unter Kontrolle einer Promotorsequenz, welche die Expression des im erfindungsgemäßen Verfahren bevorzugt verwendeten Polypeptids erlaubt. Ein bevorzugtes Beispiel für einen solchen Vektor ist pUC-D26.

Eine oben beschriebene Nukleinsäure oder ein oben beschriebener Vektor kann in einer Zelle vorliegen, wobei die Zelle mit mindestens einer Kopie einer solchen Nukleinsäure oder eines solchen Vektors transformiert ist. Vorzugsweise ist die Zelle eine prokaryontische Zelle, besonders bevorzugt eine gram-negative prokaryontische Zelle und am meisten bevorzugt eine E.coli-Zelle.

Das in dem erfindungsgemäßen Verfahren verwendete Polypeptid wird vorzugsweise als Antigen in einem immunologischen Testverfahren verwendet. Andererseits kann das Polypeptid jedoch auch als Helicase-Protein und aufgrund seiner hervorragenden antigenen Wirkung zur Herstellung eines Impfstoffs gegen eine HCV-Infektion verwendet werden.

Das erfindungsgemäße Verfahren dient zur immunologischen Bestimmung eines gegen Hepatitis C-Virus gerichteten Antikörpers in einer Probenflüssigkeit und ist dadurch gekennzeichnet, dass man die Probenflüssigkeit mit mindestens einem erfindungsgemäßen Polypeptid inkubiert und den Antikörper über eine Bindung mit dem Polypeptid nachweist. Dieses immunologische Bestimmungsverfahren kann an sich nach jedem bekannten Testformat erfolgen, z.B. in einem homogenen Immunoassay mit einer einzigen Reaktionsphase oder in einem heterogenen Immunoassay mit mehr als einer Reaktionsphase. Vorzugsweise wird ein heterogenes Testformat verwendet, bei dem das Vorhandensein des Antikörpers in Anwesenheit einer reaktiven Festphase nachgewiesen wird.

Eine Ausführungsform dieses Testformats ist das sogenannte Doppelantigen-Brückentestkonzept. Bei einem derartigen Verfahren inkubiert man die Probenflüssigkeit mit mindestens zwei in dem erfindungsgemäßen Verfahren verwendbaren Polypeptiden P₁ und P₂, wobei das Polypeptid P₁ (a) an eine Festphase gebunden ist oder (b) in einer an eine Festphase bindefähigen Form vorliegt, und das Polypeptid P₂ eine Markierungsgruppe trägt. Der Antikörper in der Probenflüssigkeit wird durch Bestimmung der Markierung in der Festphase oder/und in der flüssigen Phase, vorzugsweise in der Festphase, über einen immobilisierten, d.h. an die Festphase gebundenen Komplex nachgewiesen.

Die Testdurchführung beinhaltet vorzugsweise ein Mischen der Probenflüssigkeit mit einem gereinigten markierten Antigen P₂ sowie mit dem gereinigten festphasenseitigen Antigen P₁, um einen markierten immobilisierten Komplex aus markiertem Antigen, Antikörper und Festphasen-gebundenem Antigen zu erhalten. Gegenüber anderen Testformaten zum Nachweis von Antikörpern führen die Brückentestformate sowohl zu einer Verbesserung der Sensitivität, d.h. es werden zusätzliche Immunglobulinklassen wie etwa IgM erkannt, als auch der Spezifität, d.h. unspezifische Reaktivitäten mit Anti-IgG-Konjugat werden verringert.

Das markierte Antigen P₂ trägt eine direkt oder indirekt nachweisbare Markierungsgruppe wie oben beschrieben. Das festphasenseitige Antigen P₁ kann z.B. über einen bifunktionellen Spacer direkt an die Festphase gebunden sein. Vorzugsweise ist P₁ jedoch ein in der flüssigen Phase vorliegendes Konjugat aus einem in dem erfindungsgemäßen Verfahren verwendbaren Polypeptid und einem Reaktionspartner eines spezifischen Bindungssystems. Der andere Reaktionspartner des spezifischen Bindungssystems liegt an der Festphase gebunden vor. Beispiele für solche spezifischen Bindungssysteme sind Biotin/Avidin, Biotin/Strepatavidin, Biotin/Antibiotin, Hapten/Antihapten, Kohlenhydrat/Lectin und Antikörper bzw. Antikörperfragment und Antikörper gegen diesen Antikörper bzw. gegen das Antikörperfragment. Vorzugsweise liegt das Antigen P₁ in Form eines Biotin-Konjugats vor.

Das Polypeptid-Antigen wird in einem derartigen Doppelantigen-Brückentest vorzugsweise in löslicher Form eingesetzt, um Leerwerterhöhungen und eine ungünstige Signal/Rausch-Relation aufgrund von Aggregationen des Antigens zu vermeiden. Hierzu wird das Antigen entweder in geeigneten Expressionssystemen bereits löslich exprimiert, oder nach Expression in löslicher Form auf bekannte Weise in vitro renaturiert. Weiterhin kann zur Vermeidung der Ausbildung von kovalent vernetzten Molekülaggregaten der immunologische Test unter milden reduzierenden Bedingungen (Zusatz von milden reduzierenden Reagenzien, vorzugsweise von Sulfydrylreagenzien, bevorzugt DTT (Dithiothreitol) oder DTE (Dithioerythritol) im Konzentrationsbereich von 1 mmol/l bis 25 mmol/l) durchgeführt werden oder/und bevorzugt ein an Sulfhydrylgruppen kovalent modifiziertes Antigen oder/und bevorzugt ein Antigen mit mindestens partiell substituierten Cysteinresten verwendet werden. Eine ausführliche Beschreibung des Brückentestformats findet sich in EP-A-0 280 211. Auf diese Offenbarung wird hiermit Bezug genommen. Die reduzierenden Bedingungen sind vor allem wesentlich für die verbesserte Sensitivität (Konverterkennung) und die verbesserte Stabilität des NS3-Antigens.

Das in dem erfindungsgemäßen Verfahren verwendbare Polypeptid kann aber auch in anderen Testformaten eingesetzt werden. Ein Beispiel hierfür ist ein indirekter Immunoassay zur Erkennung von spezifischem Immunglobulin durch Bindung an ein wandseitiges spezifisches Antigen und indirekten Nachweis über ein Konjugat mit einem zweiten Antikörper. Bei dieser Ausführungsform des erfindungsgemäßen Verfahrens inkubiert man die Probenflüssigkeit mit einem Polypeptid P₁, das (a) an eine Festphase gebunden ist oder (b) in einer an eine Festphase bindefähigen Form vorliegt, und mit einem weiteren, gegen P₁ gerichteten Antikörper, der eine Markierungsgruppe trägt. Der zu bestimmende Antikörper wird indirekt durch Bestimmung der Markierung in der Festphase oder/und in der flüssigen Phase, vorzugsweise in der Festphase nachgewiesen. Bei diesem Verfahren ist das an der Festphase durch einen immobilisierten Komplex aus markiertem Antikörper und festphasengebundenem Antigen erzeugte Signal indirekt proportional zur Konzentration von zu bestimmenden Antikörpern in der Probenflüssigkeit.

Das in dem erfindungsgemäßen Verfahren verwendete Polypeptid kann in einem Reagenz zur immunologischen Bestimmung eines gegen Hepatitis C-Virus gerichteten Antikörpers vorliegen, wobei das Reagenz mindestens ein in dem erfindungsgemäßen Verfahren verwendbares Polypeptid enthält. Wird das Reagenz in einem Doppelantigen-Brückentest verwendet, so enthält es vorzugsweise mindestens zwei Polypeptide P₁ und P₂, wobei das Polypeptid P₁ (a) an eine Festphase gebunden ist oder (b) in einer an eine Festphase bindefähigen Form vorliegt und das Polypeptid P₂ eine Markierungsgruppe trägt. Die Bindung des Polypeptids P₁ an die Festphase kann entweder durch direkte Bindung oder über ein spezifisches Bindepaar, vorzugsweise Straptavidin/Avidin und Biotin, erfolgen. Besonders bevorzugt liegt das Polypeptid P₁ in biotinylierter Form vor.

Bei Verwendung in einem indirekten Immuntest enthält das Reagenz vorzugsweise ein Polypeptid P₁, das (a) an eine Festphase gebunden ist, oder (b) in einer an eine Festphase bindefähigen Form vorliegt, und einen gegen P₁ gerichteten Antikörper, der eine Markierungsgruppe trägt. Die Herstellung von gegen das verwendete Polypeptid gerichteten Antikörpern erfolgt auf bekannte Weise durch Immunisierung von Versuchstieren mit dem entsprechenden Antigen und Gewinnung von polyklonalen Antiseren aus dem Versuchstier. Alternativ kann nach dem Verfahren von Köhler und Milstein oder einer Weiterentwicklung dieses Verfahrens ein monoklonaler Antikörper gegen das Antigen hergestellt werden. Anstelle eines vollständigen Antikörpers können auch Antikörperfragmente oder Antikörperderivate eingesetzt werden.

Ein weiteres Anwendungsgebiet der in dem erfindungsgemäßen Verfahren verwendeten Polypeptide ist beispielsweise die Herstellung von Impfstoffen. Hierzu werden die Polypeptide vorzugsweise in gereinigter Form hergestellt und dann in Form von injizierbaren Flüssigkeiten gebracht, wobei es sich entweder um Lösungen oder Suspensionen der Polypeptide handeln kann. Die Polypeptide können auch in Liposomen eingeschlossen sein. Weitere Bestandteile der Impfstoffe sind z.B. Wasser, Salzlösungen, Glucose oder Glycerin. Darüber hinaus enthalten die Impfstoffe geringe Mengen an Hilfssubstanzen, wie Emulgatoren, Puffersubstanzen, gegebenenfalls Adjuvantien, die die Immunantwort steigern. Die Impfstoffe werden üblicherweise parenteral durch Injektion, vorzugsweise subkutan oder intramuskulär appliziert.

Weiterhin wird die vorliegende Erfindung durch die nachfolgenden Beispiele und Sequenzprotokolle beschrieben.

Es zeigen:
SEQ ID NO. 1: die Nukleotidsequenz eines in dem erfindungsgemäßen Verfahren bevorzugt verwendeten Polypeptids,
SEQ ID NO. 2: die Aminosäuresequenz einer für ein in dem erfindungsgemäßen Verfahren bevorzugt verwendetes Polypeptid kodierenden Nukleinsäure,
SEQ ID NO. 3: die Nukleotidsequenz von Primer (1),
SEQ ID NO. 4: die Nukleotidsequenz von Primer (2),
SEQ ID NO. 5: die Nukleotidsequenz von Primer (3),
SEQ ID NO. 6: die Nukleotidsequenz von Primer (4),
SEQ ID NO. 7: die Nukleotidsequenz von Primer (5) und
SEQ ID NO. 8: die Nukleotidsequenz von Primer (6).

### BEISPIELE

### Beispiel 1

Klonierung und Expression eines Polypeptids mit den Aminosäuren 1207 bis 1488 aus dem NS3-Bereich des Hepatitis C-Virus.

Mittels PCR wurde ausgehend von dem Klon NS3 (DSM 6847) unter Verwendung der Primer (1) und (2), deren Nukleotidsequenz in SEQ ID NO. 3 und SEQ ID NO. 4 gezeigt ist, ein DNA-Fragment amplifiziert. Am 5'-Ende dieses DNA-Fragments befinden sich Sequenzen für die Klonierung (BamHI-, BspHI-, EcoRI-Restriktionsschnittstellen) sowie ein ATG-Kodon und ein AAA(Lys)-Kodon zur Steigerung der Expression. Am 3'-Ende befinden sich Restriktionsschnittstellen für HindIII und EcoRI sowie ein Stoppkodon (TTA). Der zu HCV homologe Bereich beginnt in den Primern (1) und (2) jeweils bei Nukleotid Nr. 19.

Das auf diese Weise erhaltene DNA-Fragment wurde in einen mit BamHI und HindIII geschnittenen pUC8-Vektor eingesetzt. Das resultierende Plasmid wurde als pUC-D26 bezeichnet.

Ein mit dem Plasmid pUC-D26 transformierter E.coli-Stamm JM 109 (Yanisch-Perron et al., Gene 33 (1985), 103) wurde in 100 ml Medium (L-Broth/Ampicillin) über Nacht inkubiert. Am nächsten Morgen wurde die Kultur mit 900 ml 2x L-Broth (10 g Trypton, 10 g Hefeextrakt, 5 g NaCl pro Liter)/Ampicillin in einem 3 I-Kolben verdünnt. Nach Zugabe von 2 ml Glycerin und 1 bis 2 Tropfen Silicon-Antischaumemulsion (Fa. Serva) wurde die Kultur dann bei ca. 185 rpm und 37°C für 2 h geschüttelt. Die Induktion und Antigenproduktion erfolgt durch Zugabe von 2 mmol/l des Induktors Isopropylthio-β-D-glactosid (IPTG) und weiteres Schütteln für 3 bis 4 h. Anschließend wurden die Bakterien durch Zentrifugation pelletiert und weiterverarbeitet.

Die Bakterienpellets von zwei 1 I-Kulturen wurden in 200 ml 50 mmol/l Tris-HCl, pH 8,5, 0,2 mg/ml Lysozym und 2 mmol/l Dithioerythritol (DTE) resuspendiert. Anschließend wurden EDTA (Endkonzentration: 15 mmol/l), Phenylmethylsulfonylfluorid (Endkonzentration: 1 mmol/l) und 4 mg DNase zugegeben. Die Suspension wurde einige Minuten mit einem Magnetrührer durchmischt und für 45 min bei 37°C in einem Wasserbad inkubiert.

Anschließend erfolgte eine Zugabe von Triton-X100 (Endkonzentration: 1 %) und 30 minütiges Rühren im Magnetrührer. Nach Einfrieren bei -20°C über Nacht und Auftauen wurden die Zellen mindestens 1 Stunde bei 37 °C gerührt und gegebenenfalls sonifiziert. Die Inkubation bei 37°C und/oder die Ultraschallbehandlung sollten so lange erfolgen, bis die Viskosität der Suspension mit den lysierten Zellen eindeutig abgenommen hat.

Anschließend wurde bei 35000 g und 4 °C für 20 Minuten zentrifugiert. Das resultierende Pellet wurde in 30 ml 50 mmol/l Tris-HCl, pH 8,5, 2 mmol/l DTE, 150 mmol/l EDTA und 1,5 % OGP (Octyl-β-D-glucoapyranosid, Fa. Biomol) resuspendiert. Diese Suspension wurde bei Raumtemperatur auf dem Magnetrührer intensiv für mindestens 3 Stunden gerührt und anschließend bei 35000 g und 4 °C für 20 Minuten zentrifugiert.

Das Pellet wurde in 100 ml 8 mol/l Harnstoff, 20 mmol/l Tris-HCl, pH 8,5, 2 mmol/l DTE aufgelöst und gerührt. Das nun gelöste Antigen kann bis zur Weiterverarbeitung bei -20°C eingefroren werden.

Die Aufreinigung des Proteins erfolgte durch die nachfolgend beschriebenen Chromatographieschritte, die bei Raumtemperatur durchgeführt wurden. Die Lagerung des Antigens zwischen den Chromatographieschritten erfolgte jeweils bei -20 °C.

Der erste Chromatographieschritt erfolgte an einer Q-Sepharose Fast Flow Säule (Pharmacia) mit einem 20 mmol/l Tris-HCl, pH 8,5, 8 mol/l Harnstoff, 2 mmol/l DTE-Puffer. Die Elution erfolgte mit einem NaCl-Gradienten (0 bis 0,7 mol/l). Der Durchlauf und die Fraktionen wurden durch SDS-PAGE getestet. Das in dem erfindungsgemäßen Verfahren verwendete Polypeptid war im ersten Hauptpeak enthalten. Die positiven Fraktionen wurden vereinigt und über Nacht gegen ein 10faches Volumen von 4 mol/l Harnstoff, 2 mmol/l DTE, 20 mmol/l Tris-HCl, pH 7,3 dialysiert.

Für den zweiten Chromatographieschritt wurde die gleiche Säule verwendet. Der Säulenpuffer war der nach dem ersten Chromatographieschritt verwendete Dialysepuffer. Die Elution erfolgte mit einem NaCl-Gradienten (0 bis 0,5 mol/l) und anschließend mit 1 mol/l NaCl, NaOH pH 13, 4 mol/l Harnstoff.

Die positiven Fraktionen wurden vereinigt und über Nacht gegen den gleichen Puffer wie oben dialysiert.

Schließlich erfolgte eine Chromatographie an einer S-Sepharose Fast Flow (Pharmacia) Säule. Der Puffer und die Elutionsbedingungen waren gleich wie bei dem vorangehenden Chromatographieschritt.

Das Antigen weist im SDS-Polyacrylamidgel eine Größe von ca. 41 kDa auf. Die Ausbeute beträgt ca. 10 mg Antigen pro Liter Kulturmedium.

### Beispiel 2

Expression eines in dem erfindungsgemäßen Verfahren bevorzugt verwendeten Antigens im Vergleich mit anderen Antigenen aus dem HCV-NS3-Bereich.

Es wurden folgende Antigene exprimiert:
a) bevorzugt verwendetes Antigen D26 (Aminosäuren 1207 bis 1488)
b) Antigen C33 (Aminosäuren 1192 bis 1457 entsprechend EP-A-0 450 931)
c) Antigen D27 (Aminosäuren 1227 bis 1528)
d) Antigen NS-3 (Aminosäuren 1007 bis 1534)

Die Expression des Antigens NS-3 erfolgte unter Verwendung des hinterlegten Klons NS-3 (DSM 6847). Die Klonierung und Expression der Antigene C33 und D27 erfolgte entsprechend der in Beispiel 1 beschriebenen Methode. Die kodierenden Bereiche der Aminosäuren 1192 bis 1457 für C33 und der Aminosäuren 1227 bis 1528 für D27 wurden mittels PCR ausgehend von dem Plasmid pUC-N3 aus dem Klon NS-3 nach Standardmethoden amplifiziert. Für C33 wurden die Primer (3) und (4) verwendet, deren Nukleotidsequenzen in SEQ ID NO. 5 bzw. SEQ ID NO. 6 angegeben sind. Für D27 wurden die Primer (5) und (6) verwendet, deren Nukleotidsequenzen in SEQ ID NO. 7 bzw. SEQ ID NO. 8 angegeben sind.
Der zu HCV homologe Bereich beginnt in den Primern (3) und (5) mit Nukleotid Nr. 19 und in den Primern (4) und (6) mit Nukleotid Nr. 13.

Die amplifizierten DNA-Fragmente wurden nach Behandlung mit den Restriktionsenzymen BamHI und HindIII und anschließender Reinigung durch Agarosegelelektrophorese in den mit BamHI und HindIII geschnittenen Vektor pUC8 inseriert. Die beiden von den pUC-Vektoren exprimierten Antigene zeigen am N-terminalen Ende einen Bereich von 13 nicht HCV-kodierten Fremdaminosäuren (Met-Thr-Met-Ile-Thr-Asn-Ser-Arg-Gly-Ser-Ile-Met-Lys).

Anschließend wurden die Plasmide in E.coli JM109 transformiert. Bei Klonen, die ein für das C33-Antigen kodierendes DNA-Fragment erhalten, wird ein Antigen mit einem Molekulargewicht von ca. 34 kDa (SDS-PAGE) exprimiert. Bei Klonen mit einem DNA-Fragment, das für das Antigen D27 kodiert, wird eine Bande mit 48 kDa gefunden.

Der Anteil am Gesamtprotein beträgt bei C33 ca. 10 % und ist etwa genau so groß wie bei dem Polypeptid D26. Für D27 wird eine erheblich schwächere Expression im Bereich von 5 % oder weniger gefunden.

Lysate aus D26, D27 und C33 exprimierenden Klonen wurden nebeneinander im SDS-Gel aufgetrennt und auf Nitrocellulose transferiert. Nach Inkubation über Nacht mit verschiedenen HCV-positiven Seren in 1:100 Verdünnung erfolgte ein Nachweis gebundener Antikörper mittels eines Anti-human-IgG-Antikörper-Peroxidase-Konjugats und Farbreaktion mit 3,3'-Diaminobenzidin-tetrachlorid (DAB)/Wasserstoffperoxid.

Bei stark positiven Seren ist bei allen HCV-Antigensegmenten eine gute Reaktivität zu finden. Im Fall von schwach positiven NS3-HCV-Seren zeigt das C33-Antigen im Vergleich zu dem D26-Antigen in einigen Fällen die gleiche und in anderen Fällen eine etwas schwächere Reaktivität. Bei D27 findet man jedoch eine deutlich verminderte Farbreaktion mit schwach NS3-positiven Seren.

### Beispiel 3

Untersuchung von Spezifität und Sensitivität von verschiedenen Antigenen aus der NS3-Region von HCV.

Mit dem indirekten Testkonzept wurde die Reaktivität verschiedener Antigene aus dem NS3-Bereich mit insgesamt 960 negativen Seren vergleichend bewertet. Bei dieser Bewertung wurde eine deutlich überlegene Spezifität des Antigens D26 gegenüber dem Vergleichsantigen C33 gefunden, die sich in einer erheblich geringeren Anzahl an falsch positiven Ergebnissen äußert.

| Konstrukt | falsch positive Bewertung | richtig negative Bewertung |
|---|---|---|
| C33 | 8 | 952 |
| D26 | 2 | 958 |

Weiterhin wurde mit dem indirekten Testkonzept die Reaktivität verschiedener NS3-Konstrukte mit 20 Seren mit gesichertem HCV-Status vergleichend bewertet. Bei vergleichbarer Sensitivität der Kontrukte NS3 (Aminosäuren 1007 bis 1534), C33, D26 und D27 wurde eine geringere Sensitivität des Antigens D27 gefunden.

| Konstrukt | richtig positive Bewertung | falsch negative Bewertung |
|---|---|---|
| NS3 | 20 | 0 |
| C33 | 20 | 0 |
| D26 | 20 | 0 |
| D27 | 19 | 1 |

### Beispiel 4

Untersuchung der Stabilität von Antigenen aus der NS3-Region von HCV.

Mit dem indirekten Testkonzept wurde die Stabilität der Antigene NS3 und D26 vergleichend bewertet. Es wurde eine deutlich schlechtete Stabilität des Antigens NS3 gegenüber dem in dem erfindungsgemäßen Verfahren bevorzugt verwendeten Antigen D26 gefunden. Die Stabilität der Antigene wurde nach 72 h Inkubation bei 37°C untersucht.

| Konstrukt | Proben Nr. | Signalwiederfindung |
|---|---|---|
| NS3 | 1 | < 20 % |
| | 2 | < 20 % |
| D26 | 1 | 99 % |
| | 2 | 103% |

### Beispiel 5

Untersuchung der Reaktivität des HCV-NS3-Helicase-Antigens in An- bzw. Abwesenheit von reduzierenden Reagenzien.

Es wurde der Zeitpunkt der Serokonversion anhand der Reaktivität von zu verschiedenen Zeitpunkten entnommenen Serumproben mit HCV-NS3-Helicase-Antigen unter physiologischen Bedingungen mit bzw. ohne 20 mmol/l DTT getestet. Das Testkonzept war ein Doppelantigen-Brückentest zur klassenunabhängigen Erkennung aller Immunglobuline, bei dem ein mit einer elektrochemischen Markierungsgruppe (Ruthenium-Metallkomplex) versehenes und ein an eine Festphase bindefähiges Antigen (biotinyliertes Antigen) verwendet wurde.

Das Ergebnis dieses Tests ist in der nachstehenden Tabelle gezeigt. Es ist zu erkennen, dass in Anwesenheit von 20 mmol/l DTT eine um 38 Tage frühere Erkennung der Serokonversion möglich war. Weiterhin findet man in Anwesenheit von DTT eine verbesserte Signalstärke.

**TABELLE**

| Tag der Blutentnahme | Tage nach Beginn der Blutentnahme | Reaktivität des NS3-Antigens ohne DTT (Signal/Cut off) | Reaktivität des NS3-Antigens mit DTT (Signal/Cut off) |
|---|---|---|---|
| 28.07.1988 | 0 | 0,1 | 0,1 |
| 01.08.1988 | 4 | 0,1 | 0,1 |
| 08.08.1988 | 11 | 0,1 | 0,1 |
| 11.08.1988 | 14 | 0,1 | 0,1 |
| 15.08.1988 | 18 | 0,1 | 0,1 |
| 25.08.1988 | 28 | 0,1 | 0,1 |
| 29.08.1988 | 32 | 0,1 | 1,6* |
| 14.09.1988 | 48 | 0,1 | 6,5* |
| 05.10.1988 | 69 | 1,3* | 4,8* |
| 19.10.1988 | 83 | 2,1* | 6,8* |

| | | | |
|---|---|---|---|
| * positives Signal | | | |

Weiterhin wird die Erfindung durch die folgenden Ausführungsformen beschrieben:
1. Verfahren zur Bestimmung der Serokonversion beim Nachweis eines gegen Hepatitis C-Virus gerichteten Antikörpers in einer Probenflüssigkeit, wobei man die Probenflüssigkeit mit mindestens einem Polypeptid, das Sequenzbereiche aus Hepatitis C-Virus, insbesondere aus der NS3-Region von Hepatitis C-Virus enthält, inkubiert und den Antikörper über eine Bindung mit dem Polypeptid nachweist, dadurch gekennzeichnet,
   dass man ein Polypeptid aus einem Bereich, der mindestens einen Cysteinrest enthält, verwendet und einen oder mehrere Cysteinreste kovalent modifiziert oder/und durch andere Aminosäuren ersetzt.
2. Verfahren nach Ausführungsform 1 zur Früherkennung der Serokonversion.
3. Verfahren nach Ausführungsform 1 oder 2,
   dadurch gekennzeichnet,
   dass es nach einem homogenen Immunassay mit einer einzigen Reaktionsphase oder nach einem heterogenen Immunassay mit mehr als einer Reaktionsphase erfolgt.
4. Verfahren nach einer der Ausführungsformen 1 bis 3,
   dadurch gekennzeichnet,
   dass man die Probenflüssigkeit mit zwei Polypeptiden P₁ und P₂ inkubiert,
   wobei das Polypeptid P₁ (a) an eine Festphase gebunden ist oder (b) in einer an eine Festphase bindefähigen Form vorliegt und das Polypeptid P₂ eine Markierungsgruppe trägt,
   und den Antikörper durch Bestimmung der Markierung in der Festphase oder/und in der flüssigen Phase nachweist.
5. Verfahren nach einer der Ausführungsformen 1 bis 4,
   dadurch gekennzeichnet,
   dass man die Probenflüssigkeit mit einem Polypeptid P₁, das (a) an eine Festphase gebunden ist oder (b) in einer an eine Festphase bindefähigen Form vorliegt und mit einem weiteren, gegen P₁ gerichteten Antikörper, der eine Markierungsgruppe trägt, inkubiert und den zu bestimmenden Antikörper durch Bestimmung der Markierung in der Festphase oder/und in der flüssigen Phase nachweist.
6. Verfahren nach einer der Ausführungsformen 1 bis 5,
   dadurch gekennzeichnet,
   dass die Probenflüssigkeit menschliches Serum ist.

## Patentansprüche

1. Verfahren zur immunologischen Bestimmung eines gegen Hepatitis C Virus gerichteten Antikörpers in einer Probenflüssigkeit, wobei man die Probenflüssigkeit mit mindestens einem Polypeptid, das Sequenzbereiche aus Hepatitis C-Virus, insbesondere aus der NS3-Region von Hepatitis C-Virus enthält, inkubiert und den Antikörper über eine Bindung mit dem Polypeptid nachweist,
**dadurch gekennzeichnet,**
**dass** man ein Polypeptid aus einem Bereich, der mindestens einen Cysteinrest enthält, verwendet, worin ein oder mehrere der aus Cysteinresten stammenden Sulfhydrylgruppen in kovalent modifizierter Form vorliegen oder/und ein oder mehrere Cysteinreste durch andere strukturell analoge α-Aminosäuren ersetzt sind.

2. Verfahren nach Anspruch 1,
**dadurch gekennzeichnet,**
**dass** es nach einem homogenen Immunassay mit einer einzigen Reaktionsphase oder nach einem heterogenen Immunassay mit mehr als einer Reaktionsphase erfolgt.

3. Verfahren nach Anspruche 1 oder 2,
**dadurch gekennzeichnet,**
**dass** man die Probenflüssigkeit mit zwei Polypeptiden P₁ und P₂ inkubiert,
wobei das Polypeptid P₁ (a) an eine Festphase gebunden ist oder (b) in einer an eine Festphase bindefähigen Form vorliegt und das Polypeptid P₂ eine Markierungsgruppe trägt,
und den Antikörper durch Bestimmung der Markierung in der Festphase oder/und in der flüssigen Phase nachweist.

4. Verfahren nach einem der Ansprüche 1 bis 3,
**dadurch gekennzeichnet,**
**dass** man die Probenflüssigkeit mit einem Polypeptid P₁, das (a) an eine Festphase gebunden ist oder (b) in einer an eine Festphase bindefähigen Form vorliegt und mit einem weiteren, gegen P₁ gerichteten Antikörper, der eine Markierungsgruppe trägt, inkubiert und den zu bestimmenden Antikörper durch Bestimmung der Markierung in der Festphase oder/und in der flüssigen Phase nachweist.

5. Verfahren nach einem der Ansprüche 1 bis 4,
**dadurch gekennzeichnet,**
**dass** die Probenflüssigkeit menschliches Serum ist.

6. Verfahren nach einem der Ansprüche 1 bis 5, wobei ein oder mehrere Cysteinreste durch Serin oder alpha-Aminobuttersäure ersetzt sind.

7. Verwendung von einem oder mehreren NS3-Antigen(en) worin ein oder mehrere der aus Cysteinresten stammenden Sulfhydrylgruppen in kovalent modifizierter Form vorliegen oder/und ein oder mehrere Cysteinreste durch andere strukturell analoge α-Aminosäuren ersetzt sind zur immunologischen Bestimmung eines gegen Hepatitis C Virus gerichteten Antikörpers in einer Probenflüssigkeit.

8. Reagenz zur immunologischen Bestimmung eines gegen HCV gerichteten Antikörpers, wobei das Reagenz ein Polypeptid gemäß Anspruch 1 enthält.


SEQUENZPROTOKOLL

<110> Roche Diagnostics GmbH

<120> Verfahren zur verbesserten Früherkennung der
      Serokonversion eines gegen Hepatitis C-Virus
      gerichteten Antikörpers

<130> 10791PEP2

<140> 03001660.4
<141> 2003-01-24

<150> DEP4428705.4
<151> 1994-08-12

<160> 9

<170> PatentIn Ver. 2.1

<210> 1
<211> 885
<212> DNA
<213> Hepatitis C virus

<220>
<221> CDS
<222> (1)..(885)

<400> 1
atg acc atg att acg aat tcc cgg gga tcc atc atg aaa tcc ccg gtg    48
Met Thr Met Ile Thr Asn Ser Arg Gly Ser Ile Met Lys Ser Pro Val
  1               5                  10                  15

ttc acg gat aac tcc tct cca ccg gta gtg ccc cag agc ttc cag gtg    96
Phe Thr Asp Asn Ser Ser Pro Pro Val Val Pro Gln Ser Phe Gln Val
             20                  25                  30

gct cac ctg cat gct ccc aca ggc agc ggc aag agc acc aag gtc ccg    144
Ala His Leu His Ala Pro Thr Gly Ser Gly Lys Ser Thr Lys Val Pro
         35                  40                  45

gct gca tac gca gct cag ggc tac aag gtg cta gtg ctc aac cct tct    192
Ala Ala Tyr Ala Ala Gln Gly Tyr Lys Val Leu Val Leu Asn Pro Ser
     50                  55                  60

gtt gct gca aca ttg ggc ttt ggt gcc tac atg tcc aag gct cat ggg    240
Val Ala Ala Thr Leu Gly Phe Gly Ala Tyr Met Ser Lys Ala His Gly
 65                  70                  75                  80

atc gat cct aac atc agg acc ggg gtg aga aca att acc act ggc agc    288
Ile Asp Pro Asn Ile Arg Thr Gly Val Arg Thr Ile Thr Thr Gly Ser
                 85                  90                  95

ccc att acg tac tcc act tac ggc aag ttt ctt gcc gac ggc ggg tgc    336
Pro Ile Thr Tyr Ser Thr Tyr Gly Lys Phe Leu Ala Asp Gly Gly Cys
            100                 105                 110

gca ggg ggt gct tat gac ata ata att tgt gac gag tgc cac tcc acg    384
Ala Gly Gly Ala Tyr Asp Ile Ile Ile Cys Asp Glu Cys His Ser Thr
        115                 120                 125

gat gcc aca tcc atc ttg ggc atc ggc act gtc ctt gac caa gga gag    432
Asp Ala Thr Ser Ile Leu Gly Ile Gly Thr Val Leu Asp Gln Gly Glu
    130                 135                 140

act gcg ggg gcg aaa ttg gtt gtg ttc gcc acc gcc acc cct ccg ggc    480
Thr Ala Gly Ala Lys Leu Val Val Phe Ala Thr Ala Thr Pro Pro Gly
145                 150                 155                 160

tcc gtc act gtg ccc cat ccc aac att gag gag gtt gct cta tcc acc     528
Ser Val Thr Val Pro His Pro Asn Ile Glu Glu Val Ala Leu Ser Thr
                165                 170                 175

acc gga gag atc cct ttt tac ggc aag gct atc ccc ctt gag gta atc     576
Thr Gly Glu Ile Pro Phe Tyr Gly Lys Ala Ile Pro Leu Glu Val Ile
            180                 185                 190

aag ggg ggg aga cat ctc atc ttc tgt cat tca aag agg aag tgc gat     624
Lys Gly Gly Arg His Leu Ile Phe Cys His Ser Lys Arg Lys Cys Asp
        195                 200                 205

gag ctc gcc aca aag ctg gtc gca atg ggc atc aat gcc gtg gcc tac     672
Glu Leu Ala Thr Lys Leu Val Ala Met Gly Ile Asn Ala Val Ala Tyr
    210                 215                 220

tac cgc ggt ctt gac gtg tcc gtc atc ccg acc agc ggt gat gtt gtc     720
Tyr Arg Gly Leu Asp Val Ser Val Ile Pro Thr Ser Gly Asp Val Val
225                 230                 235                 240

gtc gtg gca acc gac gcc ctc atg acc ggc tat acc ggc gac ttc gac     768
Val Val Ala Thr Asp Ala Leu Met Thr Gly Tyr Thr Gly Asp Phe Asp
                245                 250                 255

tcg gtg ata gac tgc aac acg tgt gtc act cag aca gtc gat ttc agc     816
Ser Val Ile Asp Cys Asn Thr Cys Val Thr Gln Thr Val Asp Phe Ser
            260                 265                 270

ctt gac cct acc ttc acc att gag acg acc aca ctt ccc cag gat gct     864
Leu Asp Pro Thr Phe Thr Ile Glu Thr Thr Thr Leu Pro Gln Asp Ala
        275                 280                 285

gtc tcc cgc act caa cga cgg                                         885
Val Ser Arg Thr Gln Arg Arg
    290                 295


<210> 2
<211> 295
<212> PRT
<213> Hepatitis C virus

<400> 2
Met Thr Met Ile Thr Asn Ser Arg Gly Ser Ile Met Lys Ser Pro Val
1               5                   10                  15

Phe Thr Asp Asn Ser Ser Pro Pro Val Val Pro Gln Ser Phe Gln Val
            20                  25                  30

Ala His Leu His Ala Pro Thr Gly Ser Gly Lys Ser Thr Lys Val Pro
        35                  40                  45

Ala Ala Tyr Ala Ala Gln Gly Tyr Lys Val Leu Val Leu Asn Pro Ser
    50                  55                  60

Val Ala Ala Thr Leu Gly Phe Gly Ala Tyr Met Ser Lys Ala His Gly
65                  70                  75                  80

Ile Asp Pro Asn Ile Arg Thr Gly Val Arg Thr Ile Thr Thr Gly Ser
                85                  90                  95

Pro Ile Thr Tyr Ser Thr Tyr Gly Lys Phe Leu Ala Asp Gly Gly Cys
            100                 105                 110

Ala Gly Gly Ala Tyr Asp Ile Ile Ile Cys Asp Glu Cys His Ser Thr
        115                 120                 125

Asp Ala Thr Ser Ile Leu Gly Ile Gly Thr Val Leu Asp Gln Gly Glu

130                 135                 140

Thr Ala Gly Ala Lys Leu Val Val Phe Ala Thr Ala Thr Pro Pro Gly
145                 150                 155                 160

Ser Val Thr Val Pro His Pro Asn Ile Glu Glu Val Ala Leu Ser Thr
                165                 170                 175

Thr Gly Glu Ile Pro Phe Tyr Gly Lys Ala Ile Pro Leu Glu Val Ile
            180                 185                 190

Lys Gly Gly Arg His Leu Ile Phe Cys His Ser Lys Arg Lys Cys Asp
        195                 200                 205

Glu Leu Ala Thr Lys Leu Val Ala Met Gly Ile Asn Ala Val Ala Tyr
    210                 215                 220

Tyr Arg Gly Leu Asp Val Ser Val Ile Pro Thr Ser Gly Asp Val Val
225                 230                 235                 240

Val Val Ala Thr Asp Ala Leu Met Thr Gly Tyr Thr Gly Asp Phe Asp
                245                 250                 255

Ser Val Ile Asp Cys Asn Thr Cys Val Thr Gln Thr Val Asp Phe Ser
            260                 265                 270

Leu Asp Pro Thr Phe Thr Ile Glu Thr Thr Thr Leu Pro Gln Asp Ala
        275                 280                 285

Val Ser Arg Thr Gln Arg Arg
    290                 295


<210> 3
<211> 40
<212> DNA
<213> Künstliche Sequenz

<220>
<223> Beschreibung der künstlichen Sequenz: Primer

<400> 3
aagggatcca tcatgaaatc cccggtgttc acggataact                          40


<210> 4
<211> 39
<212> DNA
<213> Künstliche Sequenz

<220>
<223> Beschreibung der künstlichen Sequenz: Primer

<400> 4
gggaagcctt aattcttacc gtcgttgagt gcgggagac                           39


<210> 5
<211> 39
<212> DNA
<213> Künstliche Sequenz

<220>
<223> Beschreibung der künstlichen Sequenz: Primer

<400> 5
gagggatcca tcatgaaagc ggtggacttt atccctgtg                           39

<210> 6
<211> 33
<212> DNA
<213> Künstliche Sequenz

<220>
<223> Beschreibung der künstlichen Sequenz: Primer

<400> 6
gagaagcttt taacacgtgt tgcagtctat cac                                33


<210> 7
<211> 39
<212> DNA
<213> Künstliche Sequenz

<220>
<223> Beschreibung der künstlichen Sequenz: Primer

<400> 7
gagggatcca tcatgaaaca cctgcatgct cccaccggc                          39


<210> 8
<211> 33
<212> DNA
<213> Künstliche Sequenz

<220>
<223> Beschreibung der künstlichen Sequenz: Primer

<400> 8
gagaagcttt taataccaag cacagcctgc gtc                                33


<210> 9
<211> 13
<212> PRT
<213> Künstliche Sequenz

<220>
<223> Beschreibung der künstlichen Sequenz: nicht
      HCV-kodierte Fremdaminosäuren am N-Terminus  von
      durch pUC-Vektoren exprimierten Antigenen

<400> 9
Met Thr Met Ile Thr Asn Ser Arg Gly Ser Ile Met Lys
  1               5                   10